# EUROPEAN PATENT APPLICATION

(11) **EP 3 279 713 A1**
(43) Date of publication of application: **07.02.2018**
(21) Application number: 16772663.7
(22) Date of filing: 25.03.2016
(51) Int. Cl.: G02B 21/26, C12M 1/34, G02B 21/36

(54) **OBSERVATION APPARATUS**

(30) Priority: 31.03.2015 JP 2015072980
(71) Applicant: Olympus Corporation, Hachioji-shi Tokyo 192-8507 (JP)
(72) Inventor: HIRATA, Tadashi, Tokyo 192-8507 (JP); TAKAHASHI, Shintaro, Tokyo 192-8507 (JP); IGA, Yasunobu, Tokyo 192-8507 (JP); TAKIMOTO, Shinichi, Tokyo 192-8507 (JP)
(74) Representative: Schicker, Silvia
(86) International application number: PCT/JP2016/059694
(87) International publication number: WO 2016/158782

(57) **Abstract**

It is possible to observe imaging subjects, such as cells or the like, without causing an increase in the apparatus size. Provided is an observation apparatus (1) including: a flat-plate-like stage (3) formed of an optically transparent material on which a container (2) accommodating a sample (P) is placed; a deflecting member (8) that is disposed below the stage (3) and that deflects light coming from the sample (P) on the stage (3) into a substantially horizontal direction; an objective lens (9) that collects the light deflected by the deflecting member (8); and an image-acquisition device (10) that captures the light collected by the objective lens (9).

## Description

### {Technical Field}

The present invention relates to an observation apparatus.

### {Background Art}

In the related art, there is a known observation apparatus with which the state of cells is observed while culturing the cells in an incubator (for example, see Patent Literature 1). In this observation apparatus, an objective lens that collects light coming from the cells, which are adhered to a bottom surface of a culturing container, is disposed below the culturing container so as to face the bottom surface of the culturing container and so that the optical axis thereof is arranged in the vertical direction, and an image-capturing optical system that includes an image-acquisition device that captures the light coming from the cells and being collected by the objective lens is disposed below the objective lens.

### {Citation List}

### {Patent Literature}

{PTL 1} Japanese Unexamined Patent Application, Publication No. 2005-326495

### {Summary of Invention}

### {Technical Problem}

Because of this, with the observation apparatus of Patent Literature 1, there is a problem in that the height thereof needs to be high enough to accommodate the length of the objective lens and the image-capturing optical system that is disposed below the objective lens, including lenses, the image-acquisition device, and so forth, and thus, it is difficult to achieve a size reduction.

The present invention has been conceived in light of the above-described circumstances, and an object thereof is to provide an observation apparatus with which it is possible to observe imaging subjects, such as cells or the like, without causing an increase in the apparatus size.

### {Solution to Problem}

In order to achieve the above-described object, the present invention provides the following solutions.

An aspect of the present invention provides an observation apparatus including: a flat-plate-like stage on which a container accommodating a sample is placed and through which light can pass; a deflecting member that is disposed below the stage and that deflects light coming from the sample on the stage into a substantially horizontal direction; an objective lens that collects the light deflected by the deflecting member; and an image-acquisition device that captures the light collected by the objective lens.

With this aspect, by placing the container accommodating the sample on the stage and by irradiating the sample with the illumination light, the light that has passed through or that has been reflected by the sample passes through the stage downward, thus being deflected by the deflecting member. Because the objective lens in which the optical axis thereof is arranged in a substantially horizontal direction is placed in the deflection direction, the light coming from the sample that has been deflected is collected by the objective lens and is captured by the image-acquisition device.

In this case, by arranging the optical axis of the objective lens in the substantially horizontal direction, with regard to the space below the stage, it suffices to have a height that corresponds to the diameter of the objective lens. In other words, by placing the objective lens so that the optical axis thereof points in the substantially horizontal direction, it is also possible to place the image-acquisition device, which captures the light collected by the objective lens, on the extension of the optical axis of the objective lens. Therefore, it is possible to reduce the thickness by reducing the height-direction size. With a thin observation apparatus, it is possible to accommodate the observation apparatus in an incubator together with the container installed on the stage, and thus, it is possible to observe the state of cells while culturing the cells.

In the above-described aspect, the objective lens may be provided with one or more movable lenses that can be moved in optical-axis directions thereof, and a focal-point adjusting mechanism that may move the movable lenses in the optical-axis directions is provided.

By doing so, when the focal-point adjusting mechanism is actuated, the one or more movable lenses that constitute the objective lens are moved in the optical-axis direction, which changes the distance between the objective lens and the deflecting member, and thus, it is possible to move the focal position of the objective lens, which is disposed above the stage, in top-to-bottom directions. Specifically, with a conventional observation apparatus in which the optical axis of the objective lens is arranged in the vertical direction, the focal position is adjusted by moving objective lens in the top-to-bottom directions, and this configuration requires a greater height, whereas, with this aspect, it is possible to adjust the focal position without increasing the height.

The above-described aspect may be provided with: an image-capturing unit provided with the deflecting member, the objective lens, and the image-acquisition device; and a moving mechanism that moves the image-capturing unit in the direction parallel to the stage.

By doing so, it is possible to ensure a large observation area by moving the image-capturing unit in the horizontal directions by actuating the moving mechanism even if the viewing-field area of the image-capturing unit itself is small. In this case also, it is possible to employ a moving mechanism having a form that extends in the horizontal directions, and thus, it is possible to achieve a thickness reduction without increasing the height.

In the above-described aspect, the stage may be formed of an optically transparent material, and a housing in which the stage serves as a top plate, and that accommodates the image-capturing unit and the moving mechanism in an airtight state may be provided.

By doing so, even if the observation apparatus is accommodated in an incubator together with the container, the interior of which has high humidity, over a long period of time, the housing in the airtight state prevents moisture from coming into contact with the image-capturing unit and the moving mechanism, and thus, it is possible to keep these devices in sound states.

The above-described aspect may be provided with a transmitting portion that transmits an image acquired by the image-acquisition device to the exterior.

By doing so, it is possible to observe the state of the sample over a long period of time by using images that are transmitted from the transmitting portion in a state in which the container is placed on the stage without having to touch the container. For example, because images acquired by the image-acquisition device are transmitted outside the incubator by the transmitting portion when culturing is started after accommodating the container in the incubator in the state in which the container is placed on the stage, it is possible to observe, over a long period of time from the exterior, the state of the sample during culturing without having to take the container out of the incubator.

### {Advantageous Effects of Invention}

The present invention affords an advantage in that it is possible to observe imaging subjects, such as cells or the like, without causing an increase in the apparatus size.

### {Brief Description of Drawings}

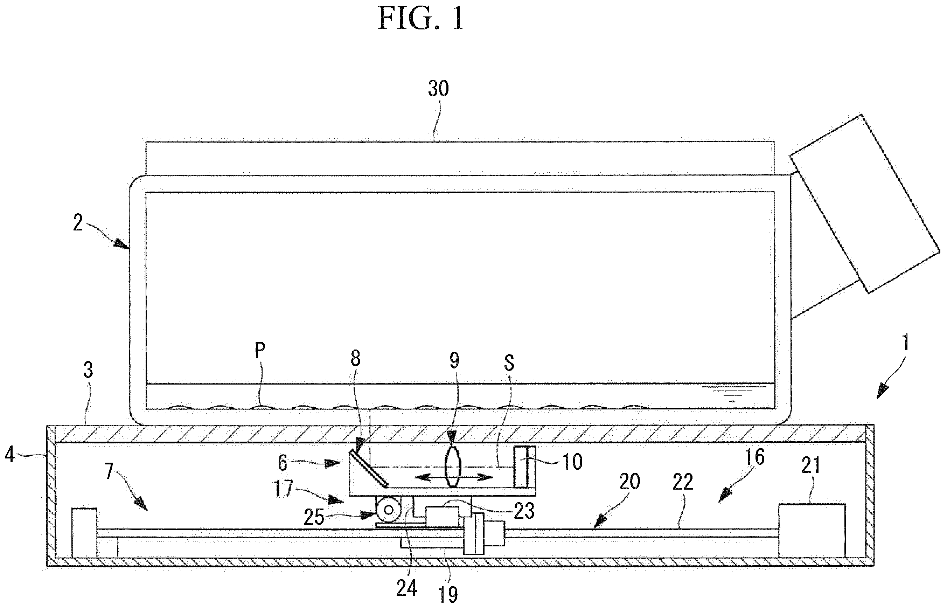

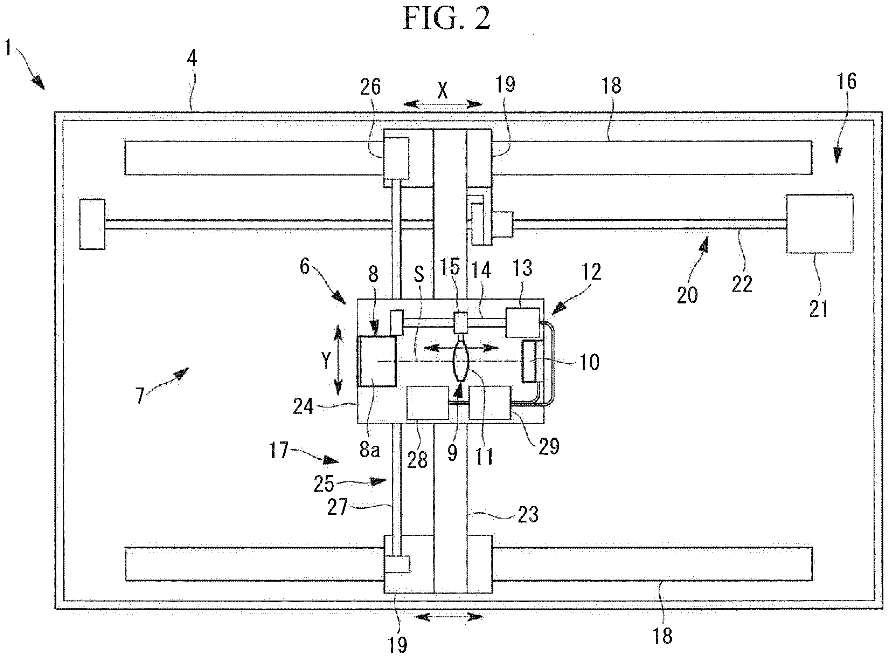

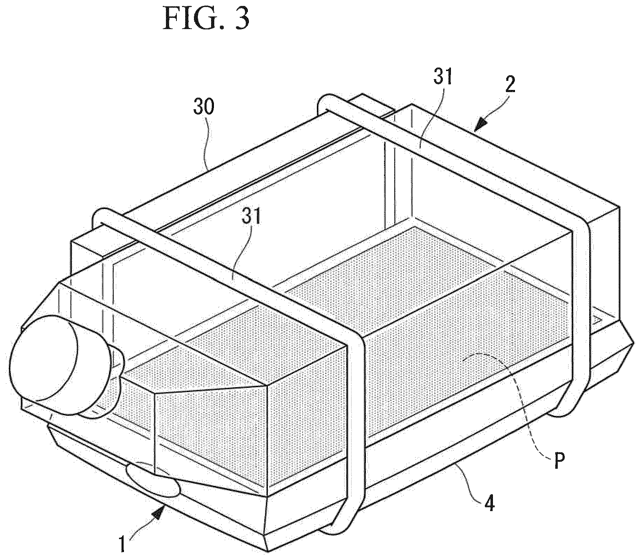

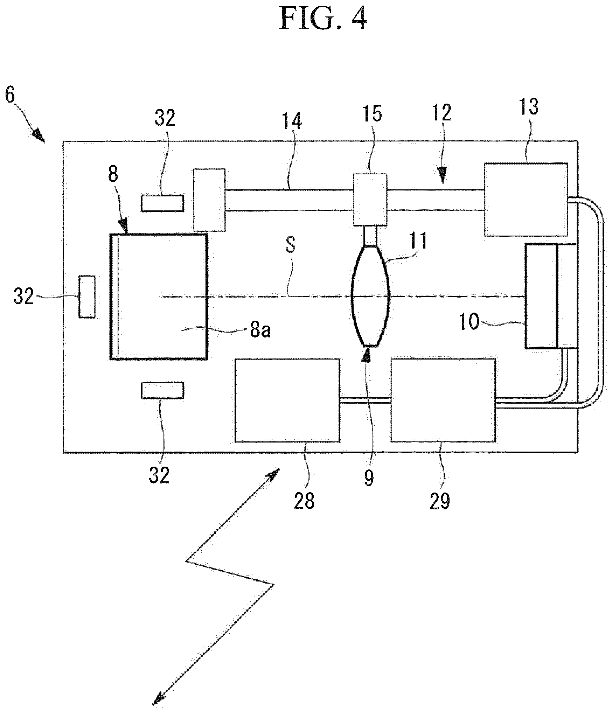

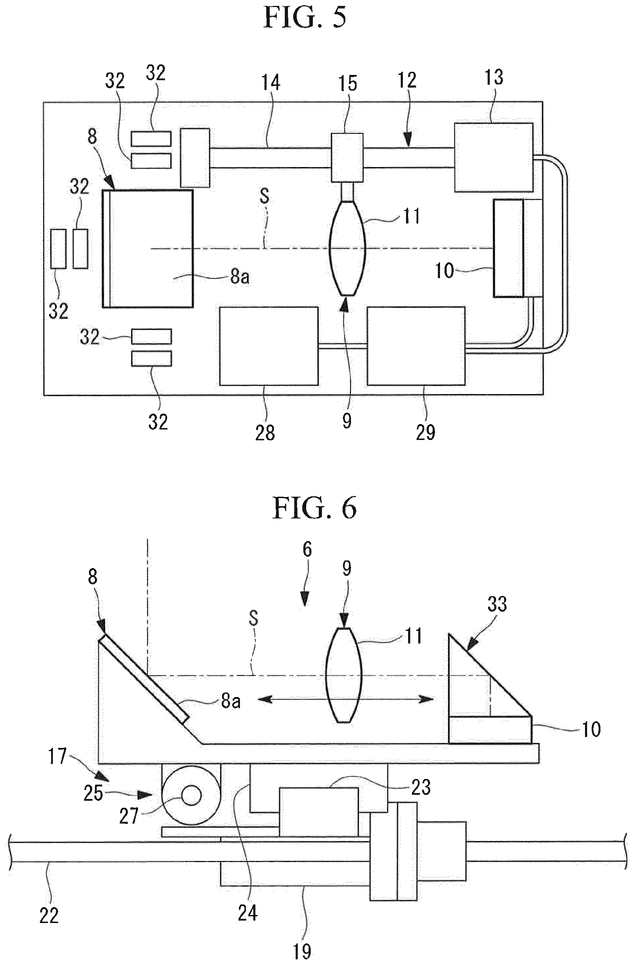

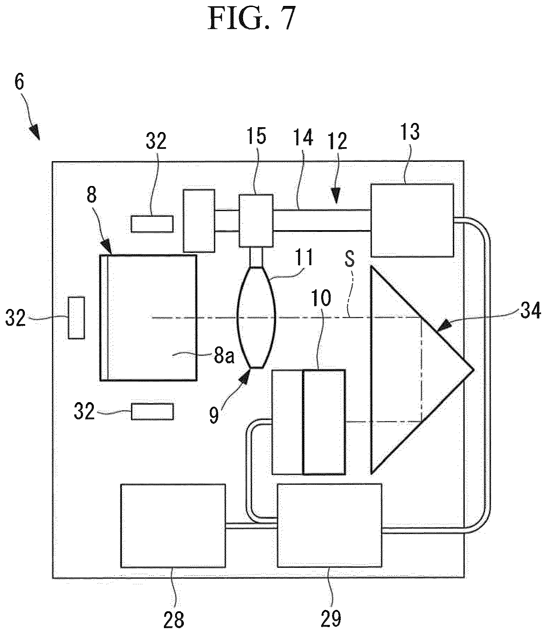

{Fig. 1} Fig. 1 is a longitudinal sectional view showing a state in which a container is installed in an observation apparatus according to an embodiment of the present invention.
{Fig. 2} Fig. 2 is a plan view showing the internal structure of the observation apparatus in Fig. 1.
{Fig. 3} Fig. 3 is a perspective view showing an example of another way of attaching an illumination apparatus to a container installed in the observation apparatus in Fig. 1.
{Fig. 4} Fig. 4 is a magnified plan view of an image-capturing unit, showing a modification of the observation apparatus in Fig. 1.
{Fig. 5} Fig. 5 is a magnified plan view of an image-capturing unit, showing another modification of the observation apparatus in Fig. 1.
{Fig. 6} Fig. 6 is a magnified side view of an image-capturing unit, showing another modification of the observation apparatus in Fig. 1.
{Fig. 7} Fig. 7 is a magnified plan view of an image-capturing unit, showing another modification of the observation apparatus in Fig. 1.

### {Description of Embodiment}

An observation apparatus 1 according to an embodiment of the present invention will be described below with reference to the drawings.

As shown in Fig. 1, the observation apparatus 1 according to this embodiment is provided with: a housing 4 in which an optically transparent stage 3 on which a container 2 accommodating a sample P is placed serves as a top plate; an image-capturing unit 6 that is disposed in the housing 4; and a moving mechanism 7 that two-dimensionally moves the image-capturing unit 6 in directions parallel to the top plate.

The stage 3 is, for example, a flat glass plate, with which it is possible to make the light coming from the sample P placed on the stage 3 pass therethrough and enter into the interior of the housing 4. The housing 4 has an airtight structure so as to prevent the entry of moisture into the housing 4 from the exterior.

The image-capturing unit 6 is provided with: a mirror (deflecting member) 8 that is disposed below the stage 3 and that deflects the light that comes from above the stage 3 and that passes therethrough; an objective lens 9 that collects the light deflected by the mirror 8; and an image-acquisition device 10 that captures the light collected by the objective lens 9.

The mirror 8 has a reflecting surface 8a that is disposed at substantially 45° with respect to the stage 3 and is configured so as to deflect the light coming from the sample P at substantially 90°, thus directing the light into a substantially horizontal direction. The objective lens 9 is provided with one or more lenses (movable lenses) 11 that are disposed so as to have a substantially horizontal optical axis S so as to collect the light deflected by the mirror 8.

As shown in Fig. 2, the image-capturing unit 6 is provided with a focal-point adjusting mechanism 12 that moves the one or more lenses 11 that constitute the objective lens 9 in the directions parallel to the optical axis S. The focal-point adjusting mechanism 12 is, for example, a linear motion mechanism provided with a motor 13 and a ball screw 14, and is configured so that the ball screw 14 is rotated by actuating the motor 13, so that a nut 15 engaged with the ball screw 14 is linearly moved along the optical axis S of the objective lens 9, and so that the lenses 11 secured to the nut 15 can thus be moved along the optical axis S of the objective lens 9.

As shown in Fig. 2, the moving mechanism 7 is constituted of two linear motion mechanisms 16 and 17 that are disposed so as to be orthogonal to each other. The first linear motion mechanism 16 is provided with: a guide rail 18 secured to the housing 4; a slider 19 that is supported so as to be movable in a first horizontal direction X along the guide rail 18; and a driving mechanism 20 that moves the slider 19. The driving mechanism 20 is provided with a motor 21 and a ball screw 22.

The second linear motion mechanism 17 is provided with: a guide rail 23 secured to the slider 19 of the first linear motion mechanism 16; a slider 24 that is supported so as to be movable in a second horizontal direction Y along the guide rail 23; and a driving mechanism 25 that moves the slider 24. The driving mechanism 25 is also provided with a motor 26 and a ball screw 27.

In the interior thereof, the housing 4 is provided with: a transmitting-receiving portion (transmitting portion) 28 that wirelessly transmits image signals acquired by the image-acquisition device 10 to the exterior and that receives instruction signals from the exterior; and a control portion 29 that controls the focal-point adjusting mechanism 12 and the moving mechanism 7 on the basis of the instruction signals received by the transmitting-receiving portion 28.

Examples of the instruction signals include: instruction signals for an operator or a program, which has confirmed the focusing state of an image by using the image signals that have been transmitted to the exterior from the transmitting-receiving portion 28, to actuate the focal-point adjusting mechanism 12 in one of two directions; and instruction signals for the operator who wishes to check another viewing-field area to move the image-capturing unit 6 in one of the two directions by means of the moving mechanism 7.

The operation of the thus-configured observation apparatus 1 according to this embodiment will be described below.

In order to observe cells (sample) P, during culturing, by using the observation apparatus 1 according to this embodiment, the cells are accommodated in the container 2, such as an optically transparent cell-culturing flask, and the cells are made to adhere to the bottom surface thereof.

The bottom surface is made to face downward in this state; the container 2 is placed on the stage 3 of the observation apparatus 1; the container 2 and the observation apparatus 1 are accommodated in an incubator; and culturing is started. The illumination light to be radiated onto the sample P may be emitted from a light source in the incubator, or, as shown in Fig. 1, the illumination light may be emitted from an illumination apparatus (light source) 30 that is secured to each container 2. Although the illumination apparatus 30 shown in Fig. 1 is an example that is secured to the top plate of the container 2, alternatively, as shown in Fig. 3, the illumination apparatus 30 may be secured to a side surface of the container 2. In Fig. 3, the reference sign 31 is a belt that secures the observation apparatus 1, the container 2, and the illumination apparatus 30 into a single unit.

When the illumination light is radiated onto the sample P in the container 2 from the illumination apparatus 30, the illumination light is refracted due to the shape or the refractive index of the sample P, or is dimmed due to the transmittance of the sample P, thus passing through the stage 3 in the form of transmitted light carrying information about the sample P, and enters the housing 4.

The light that has entered the housing 4 is deflected at 90° by the mirror 8, thus being collected by the objective lens 9 that has the optical axis S in the substantially horizontal direction, and is captured by the image-acquisition device 10. An acquired image is wirelessly transmitted by the transmitting-receiving portion 28 to the exterior, and thus, it is possible to perform observation outside the incubator.

The operator or the program checks, by using the image acquired by the image-acquisition device 10, whether or not the focal point of the objective lens 9 is appropriately aligned with the sample P, and transmits instruction signals for actuating the focal-point adjusting mechanism 12 in one of the two directions in the case in which the focal point and the sample P are not aligned. The transmitted instruction signals are received by the transmitting-receiving portion 28, thus actuating the focal-point adjusting mechanism 12.

When the ball screw 14 is rotated by the rotation of the motor 13, the nut 15 is moved in one of the horizontal directions in accordance with the rotating direction of the ball screw 14, the lenses 11 secured to the nut 15 are made to move in the horizontal direction, and, consequently, the focal position of the objective lens 9 is moved in the top-to-bottom direction.

In other words, the focal position is moved upward when the lenses 11 are moved in the direction toward the mirror 8, and the focal position is moved downward when the lenses 11 are moved in the direction away from the mirror 8. By doing so, it is possible to acquire a clear image by adjusting the focal position to an appropriate position.

When the operator wishes to observe a different position, the operator transmits instruction signals for moving the moving mechanism 7 in one of the two directions. The transmitted instruction signals are received by the transmitting-receiving portion 28, thus actuating the moving mechanism 7. The observation position is moved in one horizontal direction Y when the image-capturing unit 6 is moved along the second linear motion mechanism 17, and the observation position is moved in the other horizontal direction X when the second linear motion mechanism 17 and the image-capturing unit 6 are moved along the first linear motion mechanism 16. By doing so, it is possible to two-dimensionally adjust the observation position.

As has been described above, with the observation apparatus 1 according to this embodiment, because the light that has entered the housing 4 after passing through the stage 3 is deflected by the mirror 8 into the substantially horizontal direction and is collected by the objective lens 9 that has the substantially horizontal optical axis S, there is an advantage in that it is possible to keep the height of a space inside the housing 4 to a size that is about the same as the diameter of the objective lens 9.

In other words, as shown in Fig. 1, it is possible to observe the culturing state during culturing by accommodating the observation apparatus 1, in which the height thereof is kept at the minimum, in the incubator together with the container 2.

With the observation apparatus 1 according to this embodiment, because the focal position of the objective lens 9 is moved in the top-to-bottom direction by moving the one or more lenses 11 that constitute the objective lens 9 in the horizontal directions, it is possible to adjust the focal position without having to ensure a large height for the space inside the housing 4. Accordingly, there is an advantage in that it is possible to reduce the thickness of the observation apparatus 1.

With the observation apparatus 1 according to this embodiment, because the image-capturing unit 6 is two-dimensionally moved in the horizontal directions X and Y by means of the moving mechanism 7, even though the viewing-field area that can be captured at once may be small, it is possible to observe the state of the sample P over a greater area by capturing images thereof while moving the image-capturing unit 6 in the horizontal directions X and Y. In this case also, because the employed structure is such that a large observation area is ensured by moving the small image-capturing unit 6 in the horizontal directions X and Y, large optical components are not necessary, and thus, there is an advantage in that it is possible to reduce the thickness of the apparatus.

In this embodiment, although the observation apparatus 1 provided with the focal-point adjusting mechanism 12, the moving mechanism 7, the transmitting-receiving portion 28 and the control portion 29 has been described, the present invention is not limited thereto.

Specifically, in the case in which an objective lens 9 having a large depth of field is used, or in the case in which the focal-position adjustment is not necessary, the focal-point adjusting mechanism 12 may be omitted.

In the case in which the sample P is placed in a relatively small region, or in the case in which it suffices to observe a specific area in a portion of the sample P, it is not necessary to provide the moving mechanism 7. In the case in which the moving mechanism 7 and the focal-point adjusting mechanism 12 are not remotely operated from the exterior, the receiving function of the transmitting-receiving portion 28 is not necessary. In addition, in the case in which the moving mechanism 7 and the focal-point adjusting mechanism 12 are not provided, the receiving function of the transmitting-receiving portion 28 and the control portion 29 are not necessary.

In the case in which a storing portion (not shown) that stores images acquired by the image-acquisition device 10 is provided in the housing 4, it is not necessary to transmit the acquired image signals to the exterior, and thus, the transmitting-receiving portion 28 may be omitted.

In the description of this embodiment, although the sample P is irradiated with the illumination light coming from the light source in the incubator or the illumination apparatus 30 secured to the container 2, alternatively, as shown in Fig. 4, light sources 32 that emit illumination light upward from below the stage 3 may be provided in the area surrounding the mirror 8.

For example, it is preferable that a plurality of LED light sources that can independently be turned on be employed as the light sources 32. Because the illumination light emitted upward from the area surrounding the mirror 8 is reflected by an inner surface of the top plate of the container 2, the illumination light passes through the sample P from diagonally thereabove, and is made incident on the mirror 8 below the stage 3, it is possible to form a shadow in an image of the sample P, as in the case of oblique illumination. Therefore, it is possible to enhance the visibility of the sample P, such as transparent cells.

In this case, as shown in Fig. 5, a plurality of light sources 32 may be disposed with spaces therebetween in radial directions centered on the mirror 8, and the light sources 32 to be turned on may be changed. By doing so, it is possible to change the entry angle of the illumination light that passes through the sample P, and thus, it is possible to enhance the visibility by changing the shadow that is formed in an image of the sample P.

In this embodiment, as shown in Fig. 6, a prism 33 that deflects the light that has passed through the objective lens 9 at 90° again may be provided. By doing so, it is possible to substantially horizontally arrange the image-acquisition device 10 and a substrate to which the image-acquisition device 10 is secured, and thus, there is an advantage in that it is not necessary to increase the height of the space inside the housing 4 even if an image-acquisition device 10 having a large image-acquisition surface is employed.
Besides the mirror 8, a deflection prism 34 having a reflecting surface may be employed as the deflecting member. As shown in Fig. 7, by additionally folding the optical path, it is possible to arrange optical components from the mirror 8 to the image-acquisition device 10 in a more compact manner.

In the description of this embodiment, although a cell-culturing flask has been described as an example of the container 2, alternatively, a petri dish, a microplate, a cell-culturing bag, or the like may be employed.

In this embodiment, although a mechanism with which the focal position is adjusted by moving the lenses 11 in the direction parallel to the optical axis S with respect to the image-acquisition device 10 has been described as an example of the focal-point adjusting mechanism 12, alternatively, the focal position may be adjusted by moving the image-acquisition device 10 in the direction parallel to the optical axis S with respect to the lenses 11. The image-acquisition device 10 and the lenses 11 may be moved in the direction parallel to the optical axis S as a single unit. By doing so, because the focal position is adjusted in a state in which a predetermined spacing is kept between the image-acquisition device 10 and the lenses 11, it is possible to configure the apparatus so that the magnifications of images to be acquired are not changed even if the focal position is adjusted.

In this embodiment, although an optically transparent flat glass plate has been described as an example of the stage 3, alternatively, a flat-plate-like light-blocking member that blocks the illumination light may be employed.

In this case, the light-blocking member is provided with an opening at a portion thereof in a circumferential direction or at a portion thereof in a radial direction, and is configured so that the light coming from the sample P travels into the interior of the housing 4 by passing through the opening. The opening is formed in a size that is smaller than the bottom surface of the container 2. By doing so, it is possible to make the interior of the housing 4 airtight by placing the container 2 on the stage 3 so that the bottom surface of the container 2 covers the opening.

In the case in which the container 2 is not placed on the stage 3, because the space inside the housing 4 and the exterior are connected by the opening, access to the optical systems, such as the objective lens 9 or the like, in the housing 4 is facilitated via the opening. By doing so, it is possible to easily exchange the optical systems, such as the objective lens 9 or the like, with lenses of different magnifications.

In addition, the opening may be provided in the optically transparent flat glass plate.

{Reference Signs List}

- 1: observation apparatus
- 2: container
- 3: stage
- 4: housing
- 6: image-capturing unit
- 7: moving mechanism
- 8: mirror (deflecting member)
- 9: objective lens
- 10: image-acquisition device
- 11: lens (movable lens)
- 12: focal-point adjusting mechanism
- 28: transmitting-receiving portion (transmitting portion)
- P: sample

## Claims

1. An observation apparatus comprising:
a flat-plate-like stage on which a container accommodating a sample is placed and through which light can pass;
a deflecting member that is disposed below the stage and that deflects light coming from the sample on the stage into a substantially horizontal direction;
an objective lens that collects the light deflected by the deflecting member; and
an image-acquisition device that captures the light collected by the objective lens.

2. An observation apparatus according to Claim 1,
wherein the objective lens is provided with one or more movable lenses that can be moved in optical-axis directions thereof, and
a focal-point adjusting mechanism that moves the movable lenses in the optical-axis directions is provided.

3. An observation apparatus according to Claim 1 or 2, further comprising:
an image-capturing unit provided with the deflecting member, the objective lens, and the image-acquisition device; and
a moving mechanism that moves the image-capturing unit in the direction parallel to the stage.

4. An observation apparatus according to Claim 3,
wherein the stage is formed of an optically transparent material, and
a housing in which the stage serves as a top plate, and that accommodates the image-capturing unit and the moving mechanism in an airtight state is provided.

5. An observation apparatus according to Claim 4, further comprising:
a transmitting portion that transmits an image acquired by the image-acquisition device to the exterior.
